# EUROPÄISCHE PATENTSCHRIFT

(11) **EP 4 342 357 B1**
(45) Veröffentlichungstag und Bekanntmachung des Hinweises auf die Patenterteilung: **16.07.2025**
(21) Anmeldenummer: 22196729.2
(22) Anmeldetag: 20.09.2022
(51) Int. Cl.: A61B 1/00

(54) **VIDEO-ENDOSKOP UND UMHÜLLUNG FÜR EIN VIDEO-ENDOSKOP SOWIE VERFAHREN ZUR ÜBERWACHUNG EINER STERILBARRIERE EINES VIDEO-ENDOSKOPS**
VIDEO ENDOSCOPE AND SHEATH FOR A VIDEO ENDOSCOPE AND METHOD FOR MONITORING A STERILE BARRIER OF A VIDEO ENDOSCOPE
ENDOSCOPE VIDÉO ET ENVELOPPE POUR ENDOSCOPE VIDÉO ET PROCÉDÉ DE SURVEILLANCE D'UNE BARRIÈRE STÉRILE D'UN ENDOSCOPE VIDÉO

(43) Veröffentlichungstag der Anmeldung: 27.03.2024
(73) Patentinhaber: Alpaka Technology UG (haftungsbeschränkt), 79211 Denzlingen (DE)
(72) Erfinder: Schlegel, Marco, 79211 Denzlingen (DE)
(74) Vertreter: Westphal, Mussgnug & Partner Patentanwälte mbB

(56) Entgegenhaltungen:
- WO-A1-2019/152991
- CN-A- 112 545 433
- US-A- 5 873 814
- US-A1- 2012 302 834
- US-B1- 8 382 665

## Beschreibung

Bekannt sind Video-Endoskope mit einem Endoskop aufweisend eine Endoskop-Optik und einem Kamerakopf aufweisend eine Kameraelektronik und eine Anzeige- und Bedieneinheit.

Nachteilig bei bekannten Systemen ist ein aufwendiges Hygienekonzept aufgrund der hohen Zahl von unterschiedlichen Komponenten, welche zum Teil autoklaviert, zum Teil chemisch gereinigt und zum Teil durch einen Einweg-Sterilüberzug vom Patienten getrennt werden. Dadurch entstehen hohe Kosten für Material und Arbeitszeit.

Beispielsweise sind als Einweg-Sterilüberzüge für Video-Endoskope Umhüllungen mit einem Hohlrohr mit einem distalen Ende und einem proximalen Ende bekannt, wobei das distale Ende mittels eines optisch transparenten Fensters geschlossen ist, wobei an dem proximalen Ende ein eine Einführöffnung aufweisender flexibler Beutel angeordnet ist. Durch die Einführöffnung kann das Endoskop eingeführt werden. Durch den zum proximalen Ende offenen Beutel können Versorgungsleitungen geführt werden. Der Kamerakopf ist durch den offenen Beutel zugänglich und muss separat gereinigt werden.

Als weiterer Stand der Technik werden die US 2012 / 302834 A1, die US 8 382 665 B1, die CN 112 545 433 A und die US 5 873 814 A genannt.

Die Aufgabe der Erfindung besteht darin, ein Video-Endoskop bereitzustellen, welches ein verbessertes Hygienekonzept ermöglicht.

Die Aufgabe wird erfindungsgemäß gelöst durch ein Video-Endoskop mit den Merkmalen des Patentanspruchs 1 sowie Verfahren zur Überwachung einer Sterilbarriere eines Video-Endoskops mit den Merkmalen des Patentanspruchs 22.

Vorteilhafte Ausgestaltungen und Weiterbildungen der Erfindung sind in den abhängigen Ansprüchen angegeben.

Das erfindungsgemäße Video-Endoskop mit einem Endoskop aufweisend eine Endoskop-Optik und einem Kamerakopf aufweisend eine Kameraelektronik und eine Anzeige- und Bedieneinheit zeichnet sich dadurch aus, dass in dem Video-Endoskop eine Vakuumpumpe zum Ansaugen von Luft aus der Umgebung des Video-Endoskops angeordnet ist. Erfindungswesentlich dabei ist, dass die Vakuumpumpe in das Video-Endoskop integriert ist und somit Luft aus der Umgebung des Video-Endoskops in das Video-Endoskop einsaugt. Eine derartige Anordnung ermöglicht es, eine Umhüllung, insbesondere sofern diese luftdicht geschlossen ist, an die Oberfläche des Video-Endoskops anzusaugen, da mittels der Vakuumpumpe Luft aus der Umgebung des Video-Endoskops und somit aus dem Zwischenraum zwischen Umhüllung und Video-Endoskop angesaugt werden kann, und damit beispielsweise eine geschlossene Sterilbarriere um das Video-Endoskop bereitzustellen, was die Aufbereitung des Video-Endoskops für Operationen vereinfacht. Eine angesaugte Umhüllung, insbesondere über dem Kamerakopf, kann dabei den Vorteil aufweisen, dass sie wenig störend ist und die Bedienung des Kamerakopfes weiterhin erlaubt.

Erfindungsgemäß weist dabei das Video-Endoskop ein Gehäuse auf, in welchem die Vakuumpumpe angeordnet ist, wobei die Ansaugöffnung für die Vakuumpumpe in der Wandung des Gehäuses angeordnet ist.

In einer bevorzugten Ausführungsform ist eine Abluftöffnung in der Wandung des Gehäuses angeordnet, welche ein Anschlusselement zu einem Abluftventil einer Umhüllung für das Video-Endoskop aufweist. Dadurch wird die Möglichkeit geschaffen, die angesaugte Luft aus dem Video-Endoskop einschließlich der Umhüllung zu entfernen.

Gemäß einer alternativen bevorzugten Ausführungsform ist ein Druckluftbehälter zum Aufnahme der angesaugten Luft vorgesehen. Die angesaugte Luft verbleibt somit in dem Video-Endoskop und kann beispielsweise zur Luftkühlung wärmekritischer Komponenten oder als zusätzliche pneumatische Energiequelle, insbesondere sowohl auf der Druck- als auch auf der Vakuum-Seite der Vakuumpumpe, verwendet werden.

Eine besonders bevorzugte Ausgestaltung der Erfindung sieht vor, dass das Video-Endoskop mindestens einen Drucksensor, beispielsweise zur Bestimmung des Umgebungsdrucks, aufweist. Mittels eines derartigen Drucksensors kann eine Überwachung der Funktion der Sterilbarriere ermöglicht werden. Weiterhin können beispielsweise ein Ansaugdruck am Patienten oder andere pneumatische Anwendungen überwacht werden.

Besonders bevorzugt weist das Video-Endoskop eine kabellose Stromversorgung, welche vorzugsweise wenigstens einen Akkumulator oder wenigstens eine Batterie umfasst, auf. Durch eine derartige Ausgestaltung kann eine druckdichte Durchführung der Stromversorgung durch eine Umhüllung entfallen.

Vorteilhafterweise weist das Video-Endoskop eine Funkschnittstelle auf. Durch eine derartige Ausgestaltung kann eine druckdichte Durchführung von Datenübertragungsleitungen durch eine Umhüllung entfallen.

Vorzugsweise weist das Gehäuse wenigstens einen Stellfuß, vorzugsweise drei Stellfüße, auf, welcher wenigstens einen Saugnapf aufweist, welcher vorzugsweise mit der Vakuumpumpe verbunden ist. Mittels der Stellfüße kann das Gehäuse auf dem Patienten sicher abgestellt werden. Der oder die Saugnäpfe können eine sichere Anordnung ermöglichen. Die Beaufschlagung des Saugnapfs oder der Saufnäpfe mittels Unterdruck durch die Vakuumpumpe kann die Standsicherheit erhöhen. Insbesondere wird dadurch eine sichere und zuverlässige Positionierung des Gehäuses direkt am Patienten ermöglicht, ohne dass ein weiterer Operateur das Video-Endoskop halten muss und ohne dass ein kostenintensiver Tragarm oder Roboter zur Positionierung des Video-Endoskops erforderlich wäre.

Gemäß einer bevorzugten Ausführungsform der Erfindung umfasst die Anzeige- und Bedieneinheit ein Touch-Display. Dieses kann sich auch bei angelegter Umhüllung, welche insbesondere angesaugt ist und sich dabei insbesondere faltenfrei auf das Touch-Display anlegen lässt, einfach und zuverlässig bedienen lassen.

Vorteilhafterweise ist die Anzeige- und Bedieneinheit von einem umlaufenden Rand umgeben, wobei in dem umlaufenden Rand wenigstens eine Ansaugöffnung der Vakuumpumpe angeordnet ist. Eine derartige Ausgestaltung kann die faltenfreie Anlage der Umhüllung an die Anzeige- und Bedieneinheit, insbesondere, wenn diese als Touch-Display ausgestaltet ist, begünstigen. Da die Anzeige- und Bedieneinheit vorteilhafterweise direkt an der Wirkstätte der Operation angeordnet ist, da der Kamerakopf direkt auf dem Patienten angeordnet werden kann, kann dies auch zu einer verbesserten Augen-Hand-Koordination führen, wenn das Livebild des Video-Endoskops direkt auf dem Anzeigeelement angezeigt wird.

Vorteilhafterweise ist ein Vakuumbeutel vorgesehen, welcher mit der Vakuumpumpe verbindbar ist und in welchem ein Spülflüssigkeitsbeutel angeordnet ist. Damit besteht die Möglichkeit, mittels der Vakuumpumpe weitere Funktionen wie beispielsweise eine Spül- oder Reinigungsfunktion zu steuern.

Vorzugsweise ist das Endoskop lösbar mit dem Kamerakopf verbunden und vorzugsweise durch mit der Vakuumpumpe erzeugten Unterdruck arretierbar. Eine derartige Ausgestaltung ermöglicht ein einfaches Auswechseln der Optik für unterschiedliche Anwendungszwecke. Eine Arretierung mittels Unterdruck kann eine zuverlässige Fixierung darstellen.

Vorzugsweise umfasst das Video-Endoskop eine Umhüllung geeignet zur Aufnahme eines Video-Endoskops mit einem Hohlrohr mit einem distalen Ende und einem proximalen Ende, wobei das distale Ende mittels eines optisch transparenten Fensters geschlossen ist, wobei an dem proximalen Ende ein eine Einführöffnung aufweisender flexibler Beutel angeordnet ist, wobei die Umhüllung ein Abluftventil aufweist. Ein derartiges **Ab**luftventil ermöglicht es, Luft aus dem Innenraum der Umhüllung ablassen zu können.

Gemäß einer bevorzugten Ausführung der Erfindung weist das **Ab**luftventil ein Anschlusselement zu einer Abluftöffnung einer Vakuumpumpe eines in der Umhüllung anordenbaren Video-Endoskops auf. Dies ermöglicht das Herausführen der Luft aus dem Innenraum der Umhüllung mittels einer innerhalb des Video-Endoskops angeordneten Vakuumpumpe.

Vorzugsweise ist das Abluftventil als Einwege-Rückschlagventil ausgebildet, um ein Rückströmen von Luft durch das Abluftventil in den Innenraum der Umhüllung vermeiden zu können.

Vorzugsweist ist die Einführöffnung des Beutels mittels eines Verschlusses verschließbar ausgebildet. Die verschließbare Einführöffnung ermöglicht das vollständige Umschließen des Video-Endoskops mit der Umhüllung und das Anlegen eines Unterdrucks zum Ansaugen der Umhüllung an die Oberfläche des Video-Endoskops insbesondere an allen Flächen, besonders bevorzugt an den Bedienelementen.

Gemäß einer bevorzugten Ausführungsform der Erfindung weist das Hohlrohr an seinem proximalen Ende einen umlaufenden Kragen auf, in welchem das Abluftventil angeordnet ist. Eine derartige Ausgestaltung ermöglicht eine robuste Anordnung des **Ab**luftventils.

Vorteilhafterweise ist das Hohlrohr aus Metall oder einem ähnlich stabilen zug- und druckfesten Material gefertigt. Dies ermöglicht eine hohe Stabilität der Umhüllung.

Vorzugsweise ist der Beutel aus einem transparenten Kunststoff gefertigt. Dies ermöglicht eine flexible Anlage an unterschiedliche Gehäuseformen des Video-Endoskops, insbesondere des Kamerakopfes und dort insbesondere an das Anzeige- und Bedienelement.

Das Ansaugen kann verbessert werden, wenn vorzugsweise in dem Hohlrohr ein Kanal zwischen dem distalen Ende und dem proximalen Ende verlaufend angeordnet ist.

Besonders bevorzugt ist das Hohlrohr der Umhüllung an dem Video-Endoskop, insbesondere an dem Gehäuse, besonders bevorzugt an dem Kamerakopf, lösbar fixierbar, vorzugsweise mittels eines mechanischen Schnellverschlusses, besonders bevorzugt eines Bajonett-Verschlusses. Dadurch kann eine zuverlässige Anordnung erreicht werden und insbesondere vermieden werden, dass sich die Umhüllung während einer Operation gegen das Video-Endoskop unerwünscht verschiebt.

Gemäß einer besonders vorteilhaften Ausgestaltung der Erfindung ist eine Abluftöffnung in der Wandung des Gehäuses des Video-Endoskops, welche ein Anschlusselement zu einem Abluftventil der Umhüllung für das Video-Endoskop aufweist, und ein Abluftventil in der Umhüllung für das Video-Endoskop angeordnet, welches ein Anschlusselement zu der Abluftöffnung des Gehäuses des Video-Endoskops aufweist, wobei bei Fixierung des Hohlrohrs an dem Video-Endoskop das Anschlusselement der **Ab**luftöffnung und das Anschlusselement des Abluftventils gekoppelt werden. Durch eine derartige Ausgestaltung kann die Handhabung des Video-Endoskops insbesondere bei der Vorbereitung der Operation vereinfacht werden.

Das erfindungsgemäße Verfahren zur Überwachung der Sterilbarriere bei einem Video-Endoskop mit einer erfindungsgemäßen Umhüllung weist folgende Schritte auf:
- Einsetzen des Video-Endoskops in die Umhüllung,
- Erzeugen eines Unterdrucks in der Umhüllung,
- Detektieren des in der Umhüllung herrschenden Drucks und Vergleichen des herrschenden Drucks mit einem vorgegebenen Schwellenwert und
- Ausgeben eines Alarmsignals bei Überschreiten des Schwellenwerts durch den herrschenden Druck.

Ein derartiges Verfahren ermöglicht auf einfache Art und Weise eine Überwachung, ob die Sterilbarriere während der Operation intakt ist, und kann zur Patientensicherheit beitragen.

Vorzugsweise wird die Umhüllung nach dem Einsetzen des Video-Endoskops in die Umhüllung verschlossen.

Gemäß einer vorteilhaften Weiterbildung wird das Video-Endoskop auf einem Patienten positioniert. Dies ermöglicht eine einfachere Handhabung.

Besonders bevorzugt weist das Video-Endoskop ein Gehäuse mit wenigstens einem Stellfuß, vorzugsweise drei Stellfüßen, aufweist, welcher wenigstens einen Saugnapf aufweist, welcher in einem weiteren Schritt an den Patienten angesaugt wird. Dadurch kann eine besondere sichere Positionierung des Video-Endoskops erreicht werden.

Vorzugsweise wird ein Alarmsignal bei Lösen des Saugnapfs von dem Patienten ausgegeben. Dadurch kann ein Operateur einen Hinweis auf eine Repositionierung des Video-Endoskops erhalten.

Ein Ausführungsbeispiel der Erfindung wird anhand der nachfolgenden Figuren ausführlich erläutert. Es zeigen
- Figur 1: einen Schnitt durch ein erstes Ausführungsbeispiel eines erfindungsgemäßen Video-Endoskops mit einer erfindungsgemäßen Umhüllung,
- Figur 2: eine perspektivische Ansicht des Video-Endoskops gemäß Figur 1,
- Figur 3: eine Ausschnittvergrößerung des Video-Endoskops gemäß Figur 2,
- Figur 4: einen Schnitt durch ein zweites Ausführungsbeispiel eines erfindungsgemäßen Video-Endoskops mit einer erfindungsgemäßen Umhüllung, und
- Figur 5: einen Schnitt durch ein drittes Ausführungsbeispiel eines erfindungsgemäßen Video-Endoskops mit einer erfindungsgemäßen Umhüllung,
- Figur 6: eine perspektivische Ansicht eines vierten Ausführungsbeispiels eines erfindungsgemäßen Video-Endoskops in einer Anwendung,
- Figur 7a: eine schematische Darstellung einer Spülvorrichtung für ein Video-Endoskop gemäß einer der Figuren 1 bis 6 und
- Figur 7b: eine schematische Darstellung eines distalen Endes des Schafts des Endoskops eines Video-Endoskops gemäß einer der Figuren 1 bis **6****.**

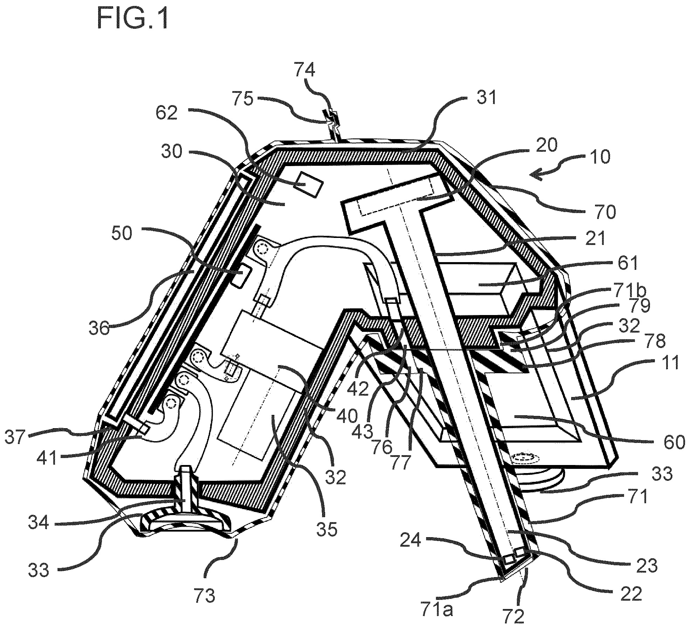

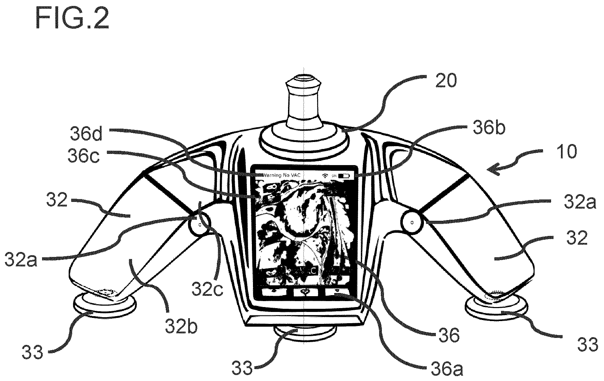

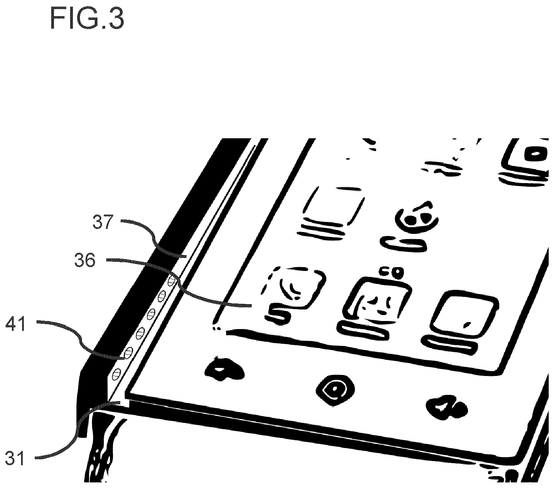

Figur 1 zeigt einen Schnitt durch ein erstes Ausführungsbeispiel eines erfindungsgemäßen Video-Endoskops 10 mit einer erfindungsgemäßen Umhüllung 70, wobei die Figuren 2 und 3 Ansichten des Video-Endoskops 10 zeigen.

Das Video-Endoskop 10 weist ein Endoskop 20 und einen Kamerakopf 30 auf. Das Endoskop 20 umfasst dabei eine Endoskop-Optik 22, welche insbesondere in einem Schaft 23 angeordnet ist. Der Kamerakopf 30 weist zumindest eine Kameraelektronik 35 und eine Anzeige- und Bedieneinheit 36 auf. In dem Schaft 23 kann weiterhin eine Endoskop-Lichtquelle 24 angeordnet sein.

Das Endoskop 20 und der Kamerakopf 30 können in einem Gehäuse 11 des Video-Endoskops 10 angeordnet sein. Es besteht alternativ die Möglichkeit, dass das Endoskop 20 und der Kamerakopf 30 lösbar miteinander verbunden sind, wobei das Endoskop 20 in einem Gehäuse 21 und der Kamerakopf 30 in einem Gehäuse 31 angeordnet sind. In diesem Fall umfasst das Gehäuse 11 des Video-Endoskops 10 das Gehäuse 21 des Endoskops 20 sowie das Gehäuse 31 des Kamerakopfs 30.

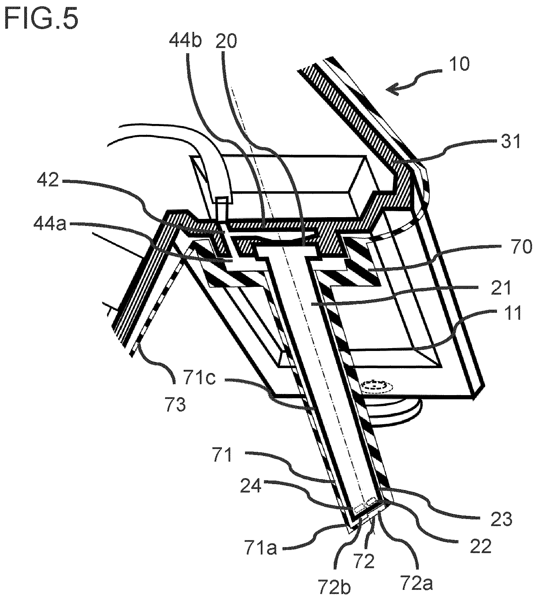

Die lösbare Verbindung kann beispielsweise mittels Unterdruck, der insbesondere mittels der Vakuumpumpe 40 erzeugt wird, erfolgen, wie in dem Ausführungsbeispiel gemäß Figur 5 dargestellt. Dazu kann in dem Gehäuse 31 eine weitere Ansaugöffnung 41 angeordnet sein, über welche ein Unterdruck in einem Zwischenraum 44b zwischen dem Gehäuse 21 des Endoskops 20 und dem Gehäuse 31 des Kamerakopfs 30 erzeugt werden kann.

In dem Video-Endoskop 10, insbesondere in dem Gehäuse 11, beispielsweise in dem Gehäuse 31 des Kamerakopfes 30, ist eine Vakuumpumpe 40 zum Ansaugen von Luft aus der Umgebung des Video-Endoskops 10 angeordnet. Eine Ansaugöffnung 41 für die Vakuumpumpe 40 kann in der Wandung des Gehäuses 11, beispielsweise in der Wandung des Gehäuses 31 des Kamerakopfes 30, angeordnet sein. Ist das Video-Endoskop 10 von einer nachfolgend detaillierter beschriebenen Umhüllung 70 umgeben, kann mittels der Vakuumpumpe 40 die Luft aus dem Zwischenraum zwischen dem Video-Endoskop 10 und der Umhüllung 70 gesaugt und die Umhüllung 70 an die Oberfläche des Video-Endoskops 10 angesaugt werden.

Die angesaugte Luft kann von der Vakuumpumpe 40 aus dem Gehäuse 11 und aus der Umhüllung 70 herausgesaugt werden. Dazu kann die in der Wandung des Gehäuses 11 bzw. 31 angeordnete Abluftöffnung 42 ein Anschlusselement 43 zu einem Abluftventil 76 der Umhüllung 70 für das Video-Endoskop 10 aufweisen.

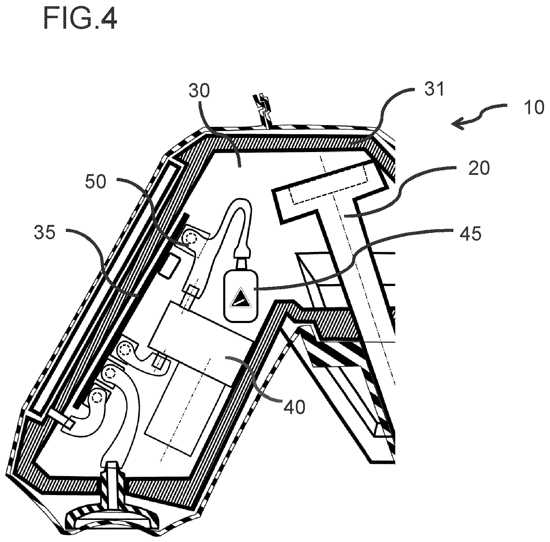

Alternativ kann, wie in Figur 4 in einem zweiten Ausführungsbeispiel des Video-Endoskops 10 dargestellt, ein Druckluftbehälter 45 zum Aufnahme der angesaugten Luft in dem Gehäuse 11, insbesondere in dem Gehäuse 31, vorgesehen sein.

Das Video-Endoskop 10 kann einen Drucksensor 50 zur Bestimmung des Umgebungsdrucks aufweisen. Dieser ist beispielsweise in dem Gehäuse 11, insbesondere in dem Gehäuse 31 des Kamerakopfes 30, derart angeordnet, dass er den Druck an der Außenfläche des Gehäuses 11 bzw. 31, insbesondere bei angelegter Umhüllung 70 in dem Zwischenbereich zwischen dem Video-Endoskop 10 und der Umhüllung 70, bestimmen kann. Das Video-Endoskop 10 kann weitere nicht dargestellte Drucksensoren aufweisen, beispielsweise in der Abluftöffnung, so dass durch Messen der Drücke oder Erfassen von Differenzdrücken Warn- oder Steuersignale erzeugt und ausgegeben werden können.

Das Video-Endoskop 10 kann eine kabellose Stromversorgung 60 aufweisen, welche vorzugsweise einen Akkumulator 61 oder eine Batterie umfasst. Der Akkumulator 61 ist vorteilhafterweise derart ausgebildet, dass ein Aufladen auf einer Ladestation, beispielsweise wenn das Video-Endoskop 10 nicht in Verwendung ist, aufladbar ist.

Weiterhin kann das Video-Endoskop 10 eine Funkschnittstelle 62 aufweisen, mittels welcher beispielsweise die von dem Endoskop 20 detektierten Bilder an einen Bildschirm im Operationssaal übertragen werden können.

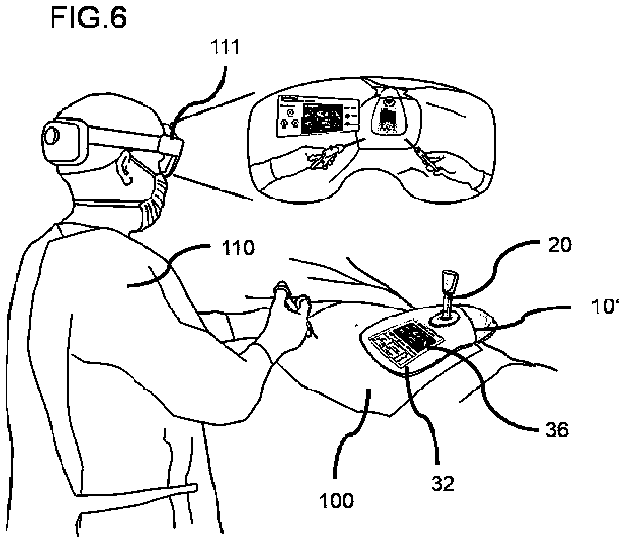

Das Gehäuse 11, im vorliegenden Ausführungsbeispiel das Gehäuse 31, kann wenigstens einen Stellfuß 32, im Ausführungsbeispiel drei Stellfüße 32, aufweisen. Jeder der Stellfüße 32 kann wenigstens einen Saugnapf 33 aufweisen. Dabei können die Saugnäpfe 33 eine Ansaugöffnung 34 aufweisen, über welche mittels der Vakuumpumpe 40 Luft angesaugt werden kann. Die Umhüllung 70 wird dadurch in die Saugnäpfe 33 eingesaugt, was die Anlage der Saugnäpfe am Körper des Patienten verbessern kann. Einer oder mehrerer der Stellfüße 32 kann zwei mittels eines Gelenks 32a, vorzugsweise eines Drehgelenks, miteinander verbundene Teilsegmente 32b, 32c aufweisen, um das Gehäuse 11 optimal an den Patienten anlegen zu können. Dies kann insbesondere direkt erfolgen, so dass dafür weder ein zusätzlicher Operateur noch ein Tragearm oder Roboter erforderlich ist (vgl. auch Figur 6).

Die Anzeige- und Bedieneinheit 36, die insbesondere auf der Außenseite des Gehäuses 11, vorliegend des Gehäuses 31, angeordnet ist, kann ein Touch-Display umfassen. Über die Anzeige- und Bedieneinheit 36 kann das Ein- und Ausschalten des Endoskops 20 erfolgen, die Bildaufnahme mittels des Endoskops 20 gestartet und beendet werden oder die Datenübertragung von dem Video-Endoskop 10 zu einem Bildschirm oder einer Verarbeitungseinheit im Operationssaal gesteuert werden. Über die Anzeige- und Bedieneinheit 36 kann beispielsweise über eine Schaltfläche 36a auch die Vakuumpumpe 40 ein- und ausgeschaltet werden. Das Touch-Display kann auch Anzeigeelemente 36b enthalten, die beispielsweise über den Ladezustand des Akkumulators 61 oder die Qualität des Funksignals informieren. Insbesondere kann ein Anzeigeelement 36c vorhanden sein, welches den Druck im Zwischenraum zwischen dem Video-Endoskop 10 und der Umhüllung 70 anzeigt. Es kann alternativ oder zusätzlich auch ein Anzeigeelement 36d vorhanden sein, dass ein Warnsignal ausgibt, falls der Druck im Zwischenraum zwischen dem Video-Endoskop 10 und der Umhüllung 70 über einen vorgegebenen Schwellenwert steigt und dadurch warnen kann, dass die Umhüllung 70 nicht dicht ist und die Sterilbarriere, die durch die Umhüllung 70 erzeugt werden soll, möglicherweise nicht zuverlässig ist. Weiterhin können weitere Druckanzeigen und/oder Warnanzeigen vorhanden sein, die beispielsweise die an einem oder vorzugsweise an sämtlichen Saugnäpfen 33 anliegenden Drücke oder basierend darauf erfolgte Auswertungen anzeigen.

Die Anzeige- und Bedieneinheit 36, insbesondere das Touch-Display, kann von einem umlaufenden Rand 37 umgeben sein. Dieser Rand 37 kann beispielsweise durch einen umlaufenden Vorsprung gebildet sein. Alternativ kann dieser Rand 37 auch Teil einer Vertiefung sein, die in dem Gehäuse 11 angeordnet ist. Dies ermöglicht eine möglichst stufenlose Oberfläche des Gehäuses 11. In dem umlaufenden Rand 37 oder im Bodenbereich unterhalb der Vertiefung kann die Ansaugöffnung 41 oder eine von mehreren Ansaugöffnungen 41 angeordnet sein, was ein faltenfreies Ansaugen der Umhüllung 70 an die Anzeige- und Bedieneinheit 36 verbessern kann.

Die erfindungsgemäße Umhüllung 70 weist ein Hohlrohr 71 mit einem distalen Ende 71a und einem proximalen Ende 71b auf, wobei das distale Ende 71a mittels eines optisch transparenten Fensters 72 geschlossen ist. Das Hohlrohr 71 kann beispielsweise aus Metall oder einem ähnlich stabilen zug- und druckfesten Material gefertigt sein. Das Fenster 72 ist vorzugsweise aus Glas oder transparentem Kunststoff gefertigt. Wird innerhalb der Umhüllung 70 ein Unterdruck erzeugt, wird vorzugsweise das Fenster 72 plan an das distale Ende des Schafts 23 angesaugt. Figur 5 zeigt eine alternative Ausgestaltung, bei welcher zwischen dem Fenster 72 und dem distalen Ende des Schafts 23 ein Abstandshalter 72a, beispielsweise durch eine Aufdickung des Fensters 72, insbesondere im Bereich vor der Endoskop-Optik 22, angeordnet sein kann, der auch bei Ansaugung der Umhüllung 70 an den Schaft 23 einen Freiraum 72b vor dem distalen Ende des Schafts 23 bewirkt, welcher eine Vakuumisolation, beispielsweise insbesondere der Endoskop-Lichtquelle 24, welche neben der Endoskop-Optik 22 angeordnet sein kann, zur Folge haben kann.

In der Regel kann der Unterdruck durch Ansaugen der Luft durch den Ringspalt zwischen der Innenseite des Hohlrohrs 71 und der Außenseite des Schafts 23 erzeugt werden. Das Ansaugen kann verbessert werden, wenn dazu in dem Hohlrohr 71 ein Kanal 71c zwischen dem distalen Ende 71a und dem proximalen Ende 71b verlaufend angeordnet ist. Zusätzlich oder alternativ kann auch ein Kanal (nicht dargestellt) in dem Schaft 23 zwischen dem distalen Ende des Schafts 23 und dem proximalen Ende des Schafts 23 angeordnet sein, durch welchen Luft vom distalen zum proximalen Ende des Schafts 23 gesaugt werden kann.

An dem proximalen Ende 71b des Hohlrohrs 71 ist ein flexibler Beutel 73 angeordnet, welcher beispielsweise aus einem transparenten Kunststoff, insbesondere einer Kunststofffolie, gefertigt sein kann. Der Beutel 73 umschließt das Hohlrohr 71 derart, dass der Innenraum des Beutels 73 mit dem Innenraum des Hohlrohrs 71 in Verbindung steht. Die Verbindung zwischen dem Beutel 73 und dem Hohlrohr 71 ist insbesondere luftdicht ausgebildet. Der Beutel 73 weist eine Einführöffnung 74 auf, welche mittels eines Verschlusses 75 insbesondere luftdicht verschließbar ausgebildet sein kann. Der Verschluss 75 kann beispielsweise als Zip-Lock-Verschluss ausgebildet sein.

Die Umhüllung 70 weist ein Abluftventil 76 auf, welches insbesondere als Einwege-Rückschlagventil ausgebildet sein kann. Das Hohlrohr 71 kann an seinem proximalen Ende 71b einen umlaufenden Kragen 78 aufweisen, in welchem das Abluftventil 76 angeordnet sein kann.

Das Abluftventil 76 kann ein Anschlusselement 77 zu der **Ab**luftöffnung 42 der Vakuumpumpe 40 des in der Umhüllung 70 anordenbaren Video-Endoskops 10 aufweisen.

Die Umhüllung 70 kann folgendermaßen verwendet werden: Durch die Einführöffnung 74 kann das Video-Endoskop 10 in die Umhüllung 70 eingeführt werden, insbesondere derart, dass das Endoskop 20 in dem Hohlrohr 71 zu liegen kommt und der Kamerakopf 30 in dem Beutel 73 angeordnet ist. Es kann eine Fixierung des Hohlrohrs 71 an dem Gehäuse 11 des Video-Endoskops 10, insbesondere des Gehäuses 31 des Kamerakopfes 30 des Video-Endoskops 10, mittels eines mechanischen Schnellverschlusses wie beispielsweise eines Bajonett-Verschlusses 79 erfolgen, welcher beispielsweise zwischen dem Kragen 78 des Hohlrohrs 71 und dem Gehäuse 11, insbesondere dem Gehäuse 31, angeordnet sein kann. Alternativ kann eine Fixierung des Hohlrohrs 71 an dem Gehäuse 11 des Video-Endoskops 10, insbesondere des Gehäuses 31 des Kamerakopfes 30 des Video-Endoskops 10, auch wie in Figur 5 dargestellt mittels eines Unterdrucks, der beispielsweise über eine zusätzliche Ansaugöffnung 41 in einem Zwischenraum 44a zwischen dem Gehäuse 31 und dem proximalen Ende des Hohlrohrs 71 erzeugt werden kann, erfolgen.

Sofern eine Abluftöffnung 42 in dem Video-Endoskop 10 und ein Abluftventil 76 in der Umhüllung 76 vorhanden, können bei Fixierung des Hohlrohrs 71 an dem Video-Endoskop 10, insbesondere an dem Gehäuse 11, vorzugsweise das Anschlusselement 43 der Abluftöffnung 42 und das Anschlusselement 77 des Abluftventils 76 gekoppelt werden, so dass bei aktivierter Vakuumpumpe 40 die durch die Vakuumpumpe 40 abgesaugte Luft durch die Abluftöffnung 42 und das Abluftventil aus dem Video-Endoskop 10 und der Umhüllung 70 herausbefördert werden kann.

Nach Einführen des Video-Endoskops 10 in die Umhüllung 70 kann die Einführöffnung 74 mittels des Verschlusses 75 verschlossen werden. Anschließend kann die Vakuumpumpe 40 aktiviert und Luft aus dem Zwischenraum zwischen dem Video-Endoskop 10 und der Umhüllung 70 gesaugt werden, um die Umhüllung 70 an die Oberfläche des Video-Endoskops 10 anzusaugen. Insbesondere wird ein Unterdruck in der Umhüllung 70 erzeugt. Der in der Umhüllung 70 herrschende Druck kann mittels des Drucksensors 50 detektiert und insbesondere kontinuierlich überwacht werden, beispielsweise durch Vergleichen des herrschenden Drucks mit einem vorgegebenen Schwellenwert oder durch Vergleichen des herrschenden Drucks mit dem Druck außerhalb der Umhüllung 70, welcher beispielsweise mittels eines weiteren nicht dargestellten Drucksensors in der Abluftöffnung 42 detektiert werden kann. Überschreitet der herrschende Druck den Schwellenwert oder sinkt die Differenz zwischen dem herrschenden Druck und dem Druck außerhalb der Umhüllung unter einen Schwellenwert, kann dies auf eine Undichtigkeit der Umhüllung 70 hinweisen, welche möglicherweise durch einen Defekt der Sterilbarriere hervorgerufen wird. Daher kann in diesem Fall ein Alarmsignal ausgegeben werden, beispielsweise durch eine Anzeige auf der Anzeige- und Bedieneinheit 36 und/oder ein akustisches Warnsignal.

Nach einer Operation kann das Video-Endoskop 10, insbesondere der Kamerakopf 30, auf eine nicht dargestellte Ladestation zum Aufladen des Akkumulators 61, gestellt werden. Dabei kann sich die Ladestation insbesondere im Operationssaal befinden.

Figur 6 zeigt ein Video-Endoskop 10', welches sich von den zuvor beschriebenen Video-Endoskopen 10 im Wesentlichen durch die Form des Gehäuses 31 des Kamerakopfes 30 unterscheidet. Im vorliegenden Ausführungsbeispiel werden der oder die Stellfüße 32 von dem Gehäuse 31 umschlossen, in welchem die Anzeige- und Bedieneinheit 36 angeordnet ist. Insbesondere kann das Gehäuse 31 lediglich einen einzigen Stellfuß 32 aufweisen. Das Video-Endoskop 10' kann, wie in Figur 6 erkennbar, direkt auf dem Körper eines Patienten 100 positioniert werden. Eine besonders sichere Positionierung ist möglich, wenn der Saugnapf 33 des Stellfußes 32 bzw. die Saugnäpfe 33 der Stellfüße 32 an den Körper des Patienten 100 angesaugt werden, insbesondere mittels der Vakuumpumpe 40. Der Druck an den Saugnäpfen 33 kann überwacht werden und bei Lösen eines Saugnapfs 33 kann ein Alarm ausgegeben werden. Ein Operateur 110 kann mittels eines klassischen Monitors (nicht dargestellt) oder bevorzugt wie dargestellt mittels eines Head-Mounted-Displays 111 das Videobild betrachten und die Kamera beispielsweise mittels Sprachbefehlen steuern. Die Sprachbefehlen können sich beispielsweise auf die Helligkeit, den Fokus und/oder den Bildausschnitt durch Zoom-in oder Zoom-out beziehen.

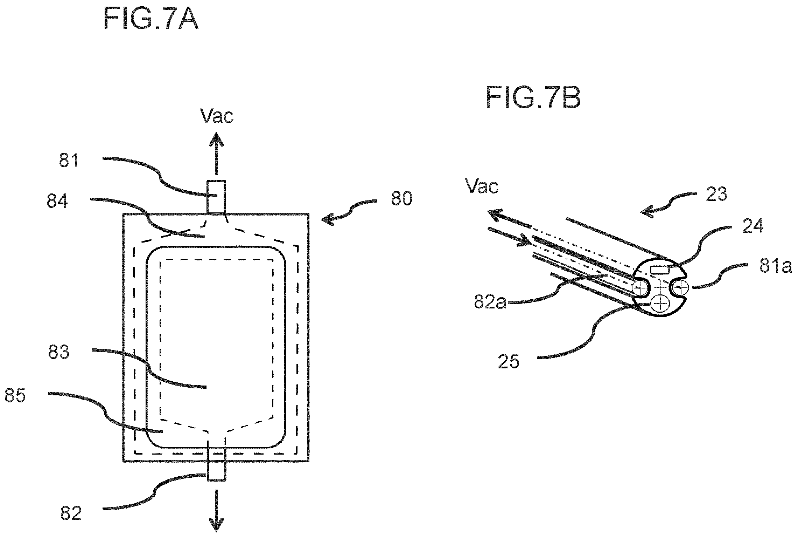

Anhand der Figuren 7a und 7b wird eine Spül-Vorrichtung 80 erläutert, welche bei den zuvor beschriebenen Video-Endoskopen 10, 10' zur Anwendung kommen kann. Die Spül-Vorrichtung 80 weist einen Spülflüssigkeitsbeutel 85 auf, in welchem eine Spülflüssigkeit 83, beispielsweise eine Kochsalzlösung, angeordnet ist. Der Spülflüssigkeitsbeutel 85 ist aus einem flexiblen Material gefertigt. Bei Druck auf den Spülflüssigkeitsbeutel 85 wird die Spülflüssigkeit 83 aus dem Spülflüssigkeitsbeutel 85 gedrückt.

Der Spülflüssigkeitsbeutel 85 ist in einem Vakuumbeutel 84 angeordnet, welcher über einen Vakuumanschluss 81 mit der Vakuumpumpe 40 verbindbar ist. Mittels der Vakuumpumpe 40 kann in dem Vakuumbeutel 84 ein Unterdruck erzeugt werden, der bewirkt, dass sich der Vakuumbeutel 84 zusammenzieht und dabei einen Druck auf den Spülflüssigkeitsbeutel 85 ausübt, wodurch die Spülflüssigkeit aus dem Spülflüssigkeitsbeutel 85 gedrückt wird. Der Spülflüssigkeitsbeutel 85 ist über einen Spülanschluss 82 mit einem Spülkanal 82a verbunden, welcher beispielsweise an der Außenseite des Schafts 23 des Endoskops 20 angebracht ist. Sofern das Endoskop 20 in der Umhüllung 70 angeordnet ist, ist der Spülkanal 82a entsprechend auf der Außenseite der Umhüllung 70 angeordnet. Zur einfachen Anordnung des Spülkanals 82a an dem Schaft 23 kann dieser beispielsweise in einer an der Außenseite des Schafts 23 angeordneten Längsnut eingelegt werden. Eine derartige Spül-Vorrichtung 80 kann auf einfache Art und Weise ein Spülen und insbesondere ein Reinigen der Endoskop-Optik 22 ermöglichen. Es kann zusätzlich ein Saugkanal 81a vorgesehen sein, welcher beispielsweise ebenfalls an der Außenseite des Schafts 23, insbesondere in einer Längsnut, angeordnet sein kann, und welcher beispielsweise mit der Vakuumpumpe 40 derart in der Verbindung steht, dass über ihn die Spülflüssigkeit 83 wieder angesaugt werden kann.

### Bezugszeichenliste

- 10: Video-Endoskop
- 10¹: Video-Endoskop
- 11: Gehäuse
- 20: Endoskop
- 21: Gehäuse
- 22: Endoskop-Optik
- 23: Schaft
- 24: Endoskop-Lichtquelle
- 30: Kamerakopf
- 31: Gehäuse
- 32: Stellfuß
- 32a: Gelenk
- 32b: Teilsegment
- 32c: Teilsegment
- 33: Saugnapf
- 34: Ansaugöffnung
- 35: Kameraelektronik
- 36: Anzeige- und Bedieneinheit
- 36a: Schaltfläche
- 36b: Anzeigeelement
- 36c: Anzeigeelement
- 36d: Anzeigeelement
- 37: Rand
- 40: Vakuumpumpe
- 41: Ansaugöffnung
- 42: Abluftöffnung
- 43: Anschlusselement
- 44a: Zwischenraum
- 44b: Zwischenraum
- 45: Druckluftbehälter
- 50: Drucksensor
- 60: Stromversorgung
- 61: Akkumulator
- 62: Funkschnittstelle
- 70: Umhüllung
- 71: Hohlrohr
- 71a: distales Ende
- 71b: proximales Ende
- 71c: Kanal
- 72: Fenster
- 72a: Abstandshalter
- 72b: Freiraum
- 73: Beutel
- 74: Einführöffnung
- 75: Verschluss
- 76: Abluftventil
- 77: Anschlusselement
- 78: Kragen
- 79: Bajonettverschluss
- 80: Spül-Vorrichtung
- 81a: Saugkanal
- 82: Spülanschluss
- 82a: Spülkanal
- 83: Spülflüssigkeit
- 84: Vakuumbeutel
- 85: Spülflüssigkeitsbeutel
- 100: Patient
- 110: Operateur
- 111: Head-Mounted-Display

## Patentansprüche

1. Video-Endoskop (10) mit einem Endoskop (20) aufweisend eine Endoskop-Optik (22) und einem Kamerakopf (30) aufweisend eine Kameraelektronik (35) und eine Anzeige- und Bedieneinheit (36),
**dadurch gekennzeichnet, dass** in dem Video-Endoskop (10) eine Vakuumpumpe (40) zum Ansaugen von Luft aus der Umgebung des Video-Endoskops (10) angeordnet ist, wobei das Video-Endoskop (10) ein Gehäuse (11) aufweist, in welchem die Vakuumpumpe (40) angeordnet ist, wobei eine Ansaugöffnung (41) für die Vakuumpumpe (40) in der Wandung des Gehäuses (11) angeordnet ist.

2. Video-Endoskop nach Anspruch 1,
**dadurch gekennzeichnet, dass** eine Abluftöffnung (42) in der Wandung des Gehäuses (11) angeordnet ist, welche ein Anschlusselement (43) zu einem Abluftventil (76) einer Umhüllung (70) für das Video-Endoskop (10) aufweist.

3. Video-Endoskop nach einem der vorhergehenden Ansprüche,
**dadurch gekennzeichnet, dass** ein Druckluftbehälter (45) zum Aufnahme der angesaugten Luft vorgesehen ist.

4. Video-Endoskop nach einem der vorhergehenden Ansprüche,
**dadurch gekennzeichnet, dass** das Video-Endoskop (10) mindestens einen Drucksensor (50), beispielsweise zur Bestimmung des Umgebungsdrucks, aufweist.

5. Video-Endoskop nach einem der vorhergehenden Ansprüche,
**dadurch gekennzeichnet, dass** das Video-Endoskop (10) eine kabellose Stromversorgung (60), welche vorzugsweise einen Akkumulator (61)oder eine Batterie umfasst, aufweist.

6. Video-Endoskop nach einem der vorhergehenden Ansprüche,
**dadurch gekennzeichnet, dass** das Video-Endoskop (10) eine Funkschnittstelle (62) aufweist.

7. Video-Endoskop nach einem der vorhergehenden Ansprüche,
**dadurch gekennzeichnet, dass** das Gehäuse (11) wenigstens einen Stellfuß (32), vorzugsweise drei Stellfüße (32), aufweist, welcher wenigstens einen Saugnapf (33) aufweist, welcher vorzugsweise mit der Vakuumpumpe (40) verbunden ist.

8. Video-Endoskop nach einem der vorhergehenden Ansprüche,
**dadurch gekennzeichnet, dass** die Anzeige- und Bedieneinheit (36) ein Touch-Display umfasst.

9. Video-Endoskop nach einem der vorhergehenden Ansprüche,
**dadurch gekennzeichnet, dass** die Anzeige- und Bedieneinheit (36) von einem umlaufenden Rand umgeben ist, wobei in dem umlaufenden Rand (37) wenigstens eine Ansaugöffnung (41) der Vakuumpumpe (40) angeordnet ist.

10. Video-Endoskop nach einem der vorhergehenden Ansprüche,
**dadurch gekennzeichnet, dass** ein Vakuumbeutel (84) vorgesehen ist, welcher mit der Vakuumpumpe (40) verbindbar ist und in welchem ein Spülflüssigkeitsbeutel (85) angeordnet ist.

11. Video-Endoskop nach einem der vorhergehenden Ansprüche,
**dadurch gekennzeichnet, dass** das Endoskop (20) lösbar mit dem Kamerakopf (30) verbunden und vorzugsweise durch mit der Vakuumpumpe (40) erzeugten Unterdruck arretierbar ist.

12. Video-Endoskop nach einem der vorhergehenden Ansprüche,
**dadurch gekennzeichnet, dass** das Video-Endoskop (10) eine Umhüllung (70) geeignet zur Aufnahme des Video-Endoskops (10) mit einem Hohlrohr (71) mit einem distalen Ende (71a) und einem proximalen Ende (71b) aufweist, wobei das distale Ende (71a) mittels eines optisch transparenten Fensters (72) geschlossen ist, wobei an dem proximalen Ende (71b) ein eine Einführöffnung (74) aufweisender flexibler Beutel (73) angeordnet ist, und wobei die Umhüllung (70) ein Abluftventil (76) aufweist.

13. Video-Endoskop nach Anspruch 12,
**dadurch gekennzeichnet, dass** das Abluftventil (76) ein Anschlusselement (77) zu einer Abluftöffnung (42) einer Vakuumpumpe (40) eines in der Umhüllung (70) anordenbaren Video-Endoskops (10) aufweist.

14. Video-Endoskop nach Anspruch 12 oder 13,
**dadurch gekennzeichnet, dass** das Abluftventil (76) als Einwege-Rückschlagventil ausgebildet ist.

15. Video-Endoskop nach einem der Ansprüche 12 bis 14,
**dadurch gekennzeichnet, dass** die Einführöffnung (74) des Beutels (73) mittels eines Verschlusses (75) verschließbar ausgebildet ist.

16. Video-Endoskop nach einem der Ansprüche 12 bis 15,
**dadurch gekennzeichnet, dass** das Hohlrohr (71) an seinem proximalen Ende (71b) einen umlaufenden Kragen (78) aufweist, in welchem das Abluftventil (76) angeordnet ist.

17. Video-Endoskop nach einem der Ansprüche 12 bis 16,
**dadurch gekennzeichnet, dass** das Hohlrohr (71) aus Metall oder einem ähnlich stabilen zug- und druckfesten Material gefertigt ist.

18. Video-Endoskop nach einem der Ansprüche 12 bis 17,
**dadurch gekennzeichnet, dass** der Beutel (73) aus einem transparenten Kunststoff gefertigt ist.

19. Video-Endoskop nach einem der Ansprüche 12 bis 18,
**dadurch gekennzeichnet, dass** in dem Hohlrohr (71) ein Kanal (71c) zwischen dem distalen Ende (71a) und dem proximalen Ende (71b) verlaufend angeordnet ist.

20. Video-Endoskop nach einem der Ansprüche 12 bis 19,
**dadurch gekennzeichnet, dass** das Hohlrohr (71) an dem Video-Endoskop (10), insbesondere an dem Gehäuse (11), besonders bevorzugt an dem Kamerakopf (30), lösbar fixierbar ist, vorzugsweise mittels eines mechanischen Schnellverschlusses, besonders bevorzugt eines Bajonett-Verschlusses (79).

21. Video-Endoskop nach Anspruch 20,
**dadurch gekennzeichnet, dass** eine Abluftöffnung (42) in der Wandung des Gehäuses (11) des Video-Endoskops (10) angeordnet ist, welche ein Anschlusselement (43) zu einem Abluftventil (76) der Umhüllung (70) für das Video-Endoskop (10) aufweist, dass ein Abluftventil (76) in der Umhüllung (70) für das Video-Endoskop (10) angeordnet ist, welches ein Anschlusselement (77) zu der Abluftöffnung (42) des Gehäuses (11) des Video-Endoskops (10) aufweist, und dass bei Fixierung des Hohlrohrs (71) an dem Video-Endoskop (10) das Anschlusselement (43) der Abluftöffnung (42) und das Anschlusselement (77) des Abluftventils (76) gekoppelt werden.

22. Verfahren zur Überwachung der Sterilbarriere bei einem Video-Endoskop (10) mit einer Umhüllung (70) nach einem der Ansprüche 12 bis 21 mit den folgenden Schritten:
- Einsetzen des Video-Endoskops (10) in die Umhüllung (70),
- Erzeugen eines Unterdrucks in der Umhüllung (70),
- Detektieren des in der Umhüllung (70) herrschenden Drucks und Vergleichen des herrschenden Drucks mit einem vorgegebenen Schwellenwert und
- Ausgeben eines Alarmsignals bei Überschreiten des Schwellenwerts durch den herrschenden Druck.

23. Verfahren nach Anspruch 22,
**dadurch gekennzeichnet, dass** das Video-Endoskop (10) auf einem Patienten (100) positioniert wird.

24. Verfahren nach Anspruch 23,
**dadurch gekennzeichnet, dass** das Video-Endoskop (10) ein Gehäuse (11) mit wenigstens einem Stellfuß (32), vorzugsweise drei Stellfüßen (32), aufweist, welcher wenigstens einen Saugnapf (33) aufweist, welcher in einem weiteren Schritt an den Patienten (100) angesaugt wird.

25. Verfahren nach Anspruch 24,
**dadurch gekennzeichnet, dass** ein Alarmsignal bei Lösen des Saugnapfs (33) von dem Patienten (100) ausgegeben wird.

## Claims

1. A video endoscope (10) having an endoscope (20) having an endoscope optical unit (22) and a camera head (30) having camera electronics (35) and a display and control unit (36),
**characterized in that** a vacuum pump (40) for suctioning air from the environment of the video endoscope (10) is arranged in the video endoscope (10), wherein the video endoscope (10) has a housing (11) in which the vacuum pump (40) is arranged, wherein a suction opening (41) for the vacuum pump (40) is arranged in the wall of the housing (11).

2. The video endoscope according to claim 1,
**characterized in that** an exhaust air opening (42) is arranged in the wall of the housing (11), which has a connecting element (43) to an exhaust air valve (76) of a sheath (70) for the video endoscope (10).

3. The video endoscope according to any one of the preceding claims,
**characterized in that** a compressed air container (45) is provided for receiving the suctioned air.

4. The video endoscope according to any one of the preceding claims,
**characterized in that** the video endoscope (10) has at least one pressure sensor (50), for example for determining the ambient pressure.

5. The video endoscope according to any one of the preceding claims,
**characterized in that** the video endoscope (10) has a wireless power supply (60), which preferably comprises a rechargeable battery (61) or a battery.

6. The video endoscope according to any one of the preceding claims,
**characterized in that** the video endoscope (10) has a radio interface (62).

7. The video endoscope according to any one of the preceding claims,
**characterized in that** the housing (11) has at least one adjustable support foot (32), preferably three adjustable support feet (32), which has at least one suction cup (33), which is preferably connected to the vacuum pump (40).

8. The video endoscope according to any one of the preceding claims,
**characterized in that** the display and control unit (36) comprises a touch display.

9. The video endoscope according to any one of the preceding claims,
**characterized in that** the display and control unit (36) is surrounded by a circumferential edge, wherein at least one suction opening (41) of the vacuum pump (40) is arranged in the circumferential edge (37).

10. The video endoscope according to any one of the preceding claims,
**characterized in that** a vacuum bag (84) is provided which can be connected to the vacuum pump (40) and in which a rinsing liquid bag (85) is arranged.

11. The video endoscope according to any one of the preceding claims,
**characterized in that** the endoscope (20) is detachably connected to the camera head (30) and lockable by means of negative pressure generated by the vacuum pump (40).

12. The video endoscope according to any one of the preceding claims,
**characterized in that** the video endoscope (10) has a sheath (70) suitable for receiving the video endoscope (10) having a hollow tube (71) with a distal end (71a) and a proximal end (71b), wherein the distal end (71a) is closed by means of an optically transparent window (72), wherein a flexible bag (73) having an insertion opening (74) is arranged at the proximal end (71b), and wherein the sheath (70) has an exhaust air valve (76).

13. The video endoscope according to claim 12,
**characterized in that** the exhaust air valve (76) has a connecting element (77) to an exhaust air opening (42) of a vacuum pump (40) of a video endoscope (10) that can be arranged in the sheath (70).

14. The video endoscope according to claim 12 or 13,
**characterized in that** the exhaust air valve (76) is designed as a one-way check valve.

15. The video endoscope according to any one of claims 12 to 14, **characterized in that** the insertion opening (74) of the bag (73) is designed to be closable by means of a closure (75).

16. The video endoscope according to any one of claims 12 to 15, **characterized in that** the hollow tube (71) has at its proximal end (71b) a circumferential collar (78) in which the exhaust air valve (76) is arranged.

17. The video endoscope according to any one of claims 12 to 16, **characterized in that** the hollow tube (71) is made of metal or a similarly stable material resistant to tension and compression.

18. The video endoscope according to any one of claims 12 to 17, **characterized in that** the bag (73) is made of a transparent plastic.

19. The video endoscope according to any one of claims 12 to 18, **characterized in that** a channel (71c) running between the distal end (71a) and the proximal end (71b) is arranged in the hollow tube (71).

20. The video endoscope according to any one of claims 12 to 19, **characterized in that** the hollow tube (71) can be detachably fixed to the video endoscope (10), in particular to the housing (11), particularly preferably to the camera head (30), preferably by means of a mechanical quick-action lock, particularly preferably a bayonet lock(79).

21. The video endoscope according to claim 20,
**characterized in that** an exhaust air opening (42) is arranged in the wall of the housing (11) of the video endoscope (10), which exhaust air opening has a connecting element (43) to an exhaust air valve (76) of the sheath (70) for the video endoscope (10), that an exhaust air valve (76) is arranged in the sheath (70) for the video endoscope (10), which exhaust air valve has a connecting element (77) to the exhaust air opening (42) of the housing (11) of the video endoscope (10), and that, when the hollow tube (71) is fixed to the video endoscope (10), the connecting element (43) of the exhaust air opening (42) and the connecting element (77) of the exhaust air valve (76) are coupled.

22. A method for monitoring the sterile barrier in a video endoscope (10) with a sheath (70) according to any one of claims 12 to 21, having the following steps:
- inserting the video endoscope (10) into the sheath (70),
- generating a negative pressure in the sheath (70),
- detecting the pressure prevailing in the sheath (70) and comparing the prevailing pressure with a predetermined threshold value, and
- outputting an alarm signal when the prevailing pressure exceeds the threshold value.

23. The method according to claim 22,
**characterized in that** the video endoscope (10) is positioned on a patient (100).

24. The method according to claim 23,
**characterized in that** the video endoscope (10) has a housing (11) having at least one adjustable support foot (32), preferably three adjustable support feet (32), which has at least one suction cup (33) which is suctioned onto the patient (100) in a further step.

25. The method according to claim 24,
**characterized in that** an alarm signal is output when the suction cup (33) detaches from the patient (100).

## Revendications

1. Endoscope (10) vidéo comprenant un endoscope (20) ayant une optique (22) d'endoscope et une tête (30) de caméra ayant une électronique (35) de caméra et une unité (36) d'affichage et de commande,
**caractérisé en ce que**, dans l'endoscope (10) vidéo est disposée une pompe (40) à vide pour aspirer de l'air de l'environnement de l'endoscope (10) vidéo, dans lequel l'endoscope (10) vidéo a un boîtier (11), dans lequel la pompe (40) à vide est disposée, dans lequel une ouverture (41) d'aspiration pour la pompe (40) à vide est disposée dans la paroi du boîtier (11).

2. Endoscope vidéo suivant la revendication **1,**
**caractérisé en ce que**, dans la paroi du boîtier (11) est disposée une ouverture (42) d'air d'échappement, qui a un élément (43) de raccordement à une soupape (76) d'air d'échappement d'une enveloppe (70) de l'endoscope (10) vidéo.

3. Endoscope vidéo suivant l'une des revendications précédentes,
**caractérisé en ce qu'**il est prévu un réservoir (45) d'air comprimé pour la réception de l'air aspiré.

4. Endoscope vidéo suivant l'une des revendications précédentes,
**caractérisé en ce que** l'endoscope (10) vidéo a au moins un capteur (50) de pression, par exemple pour la détermination de la pression ambiante.

5. Endoscope vidéo suivant l'une des revendications précédentes,
**caractérisé en ce que** l'endoscope (10) vidéo a une alimentation (60) en courant sans câble, qui comprend de préférence un accumulateur (61) ou une batterie.

6. Endoscope vidéo suivant l'une des revendications précédentes,
**caractérisé en ce que** l'endoscope (10) vidéo a une interface (62) radio.

7. Endoscope vidéo suivant l'une des revendications précédentes,
**caractérisé en ce que** le boîtier (11) a au moins un pied (32) de pose, de préférence trois pieds (32) de pose, qui a au moins un godet (33) d'aspiration, lequel communique de préférence avec la pompe (40) à vide.

8. Endoscope vidéo suivant l'une des revendications précédentes,
**caractérisé en ce que** le dispositif (36) d'affichage et de commande comprend un touch-display.

9. Endoscope vidéo suivant l'une des revendications précédentes,
**caractérisé en ce que** l'unité (36) d'affichage et de commande est entourée d'un bord faisant le tour, dans lequel au moins une ouverture (41) d'aspiration de la pompe (40) à vide est disposée dans le bord (37) faisant le tour.

10. Endoscope vidéo suivant l'une des revendications précédentes,
**caractérisé en ce qu'**il est prévu une poche (84) à vide, qui peut communiquer avec la pompe (40) à vide, et dans laquelle est disposée une poche (85) de liquide de lavage.

11. Endoscope vidéo suivant l'une des revendications précédentes,
**caractérisé en ce que** l'endoscope (20) est assemblé de manière amovible à la tête (30) de caméra et peut être arrêté de préférence par une dépression produite par la pompe (40) à vide.

12. Endoscope vidéo suivant l'une des revendications précédentes,
**caractérisé en ce que** l'endoscope (10) vidéo a une enveloppe (70) propre à la réception de l'endoscope (10) vidéo ayant un tube (71) creux à une extrémité (71a) distale et à une extrémité (71b) proximale, dans lequel l'extrémité (71a) distale est fermée au moyen d'une fenêtre (72) transparente optiquement, dans lequel une poche (73) souple ayant une ouverture (74) d'entrée est disposée à l'extrémité (71b) proximale, et dans lequel l'enveloppe (70) a une soupape (76) d'air d'échappement.

13. Endoscope vidéo suivant la revendication 12,
**caractérisé en ce que** la soupape (76) d'air d'échappement a un élément (77) de raccordement à une ouverture (42) d'air d'échappement d'une pompe (40) à vide d'un endoscope (10) vidéo pouvant être mis dans l'enveloppe (70).

14. Endoscope vidéo suivant la revendication 12 ou 13,
**caractérisé en ce que** la soupape (76) d'air d'échappement est constituée sous la forme d'un clapet anti-retour à une seule voie.

15. Endoscope vidéo suivant l'une des revendications 12 à 14,
**caractérisé en ce que** l'ouverture (74) d'introduction de la poche (73) est constituée de manière à pouvoir être fermée au moyen d'une fermeture (75).

16. Endoscope vidéo suivant l'une des revendications 12 à 15,
**caractérisé en ce que** le tube (71) creux a, à son extrémité (71b) proximale, un collet (78) faisant le tour, dans lequel la soupape (76) d'air d'échappement est disposée.

17. Endoscope vidéo suivant l'une des revendications 12 à 16,
**caractérisé en ce que** le tube (71) creux est en métal ou en un matériau semblable stable résistant à la traction et à la compression.

18. Endoscope vidéo suivant l'une des revendications 12 à 17,
**caractérisé en ce que** la poche (73) est en une matière plastique transparente.

19. Endoscope vidéo suivant l'une des revendications 12 à 18,
**caractérisé en ce que** dans le tube (71) creux, est disposé un conduit (71c) s'étendant entre l'extrémité (71a) distale et l'extrémité (71b) proximale.

20. Endoscope vidéo suivant l'une des revendications 12 à 19,
**caractérisé en ce que** le tube (71) creux peut être fixé de manière amovible à l'endoscope (10) vidéo, en particulier au boîtier (11), d'une manière particulièrement préférée à la tête (30) de la caméra, de préférence au moyen d'une fermeture mécanique rapide, d'une manière particulièrement préférée d'une fermeture (79) à baïonnette.

21. Endoscope vidéo suivant la revendication 20,
**caractérisé en ce qu'**il est disposé dans la paroi du boîtier (11) de l'endoscope (10) vidéo, une ouverture (42) pour l'air d'échappement, qui a un élément (43) de raccordement à une soupape (76) pour l'air d'échappement de l'enveloppe (70) de l'endoscope (10) vidéo, **en ce qu'**il est disposé dans l'enveloppe (70) de l'endoscope (10) vidéo une soupape (76) pour l'air d'échappement, qui a un élément (77) de raccordement à l'ouverture (42) pour l'air d'échappement du boîtier (11) de l'endoscope (10) vidéo, et **en ce que** lors de la fixation du tube (71) creux à l'endoscope (10) vidéo, l'élément (43) de raccordement de l'ouverture (42) pour l'air d'échappement et l'élément (77) de raccordement de la soupape (76) pour l'air d'échappement sont accouplés.

22. Procédé de contrôle des barrières stériles dans un endoscope (10) vidéo ayant une enveloppe (70) suivant l'une des revendications 12 à 21, comprenant les stades suivants :
- mise de l'endoscope (10) vidéo dans l'enveloppe (70),
- production d'une dépression dans l'enveloppe (70),
- détection de la pression régnant dans l'enveloppe (70) et comparaison de la pression régnante à une valeur de seuil donnée à l'avance et
- émission d'un signal d'alerte si la pression régnante dépasse la valeur de seuil.

23. Procédé suivant la revendication 22,
**caractérisé en ce que** l'on met l'endoscope (10) vidéo en position sur un patient (100).

24. Procédé suivant la revendication 23,
**caractérisé en ce que** l'endoscope (10) vidéo a un boîtier (11) ayant au moins un pied (32) de pose, de préférence trois pieds (32) de pose, qui a au moins un godet (33) d'aspiration, lequel dans un autre stade est aspiré sur le patient (100).

25. Procédé suivant la revendication 24,
**caractérisé en ce que** l'on émet un signal d'alerte, lorsque le godet (33) d'aspiration se détache du patient (100).
